Europäisches Patentamt

European Patent Office

Office européen des brevets

(19)

(11) Publication number: **0 282 330**
**A2**

## (12) EUROPEAN PATENT APPLICATION

(21) Application number: **88302149.5**

(22) Date of filing: **11.03.88**

(51) Int. Cl.⁴: **C 12 N 15/00**
C 12 N 5/00, C 12 P 21/00

(30) Priority: **13.03.87 US 25461**

(43) Date of publication of application:
**14.09.88 Bulletin 88/37**

(84) Designated Contracting States: **ES GR**

(71) Applicant: **SCHERING CORPORATION**
**2000 Galloping Hill Road**
**Kenilworth, New Jersey 07033 (US)**

(72) Inventor: **Draper, Kenneth G.**
**10 Ridgedale Avenue 29**
**Madison New Jersey 07940 (US)**

(74) Representative: **Ritter, Stephen David et al**
**Mathys & Squire 10 Fleet Street**
**London EC4Y 1AY (GB)**

(54) **Method for stimulating gene expression.**

(57) Methods, recombinant DNA constructions and stably transformed cells are described whereby enhancement of gene expression is obtained using viral transcriptional activating proteins and corresponding activation-response DNA sequences.

EP 0 282 330 A2

Bundesdruckerei Berlin

**Description**

METHOD FOR STIMULATING GENE EXPRESSION

BACKGROUND OF THE INVENTION

The present invention relates to methods for stimulating the expression of genes that have been genetically engineered into cells of various higher organisms. As such, the invention allows inducible and controllable production of proteins in a cell that is similar to the source of the heterologous protein that is being expressed. The result is the production of a protein more likely to be physiologically active in sufficient amounts for pharmaceutical purposes. Therefore, many of the problems associated with expressing heterologous genes (especially mammalian) in the usual expression systems such as bacteria, yeast and mammalian cells are lessened. These problems include the production of inactive (e.g., improperly glycosylated or improperly posttranscriptionally modified) protein in bacteria and yeast as well as inadequate gene expression in mammalian cells.

For example, the expression of eucaryotic proteins in bacterial cells has been accomplished for a variety of target genes [Proc. Natl. Acad. Sci. USA, 77, 3988 (1980)]. In the simplest case, the gene of interest is cloned into a bacterial plasmid vector, which can be maintained in an extrachromosomal episomal state once introduced into the bacterial cell. In order for the eucaryotic gene to be expressed, it must contain correctly positioned bacterial regulatory signals in its coding regions. Constructing recombinant, eucaryotic-gene-carrying plasmid vectors can be complicated and does not guarantee successful expression of the gene in bacterial cells. In the case of a gene whose expression requires enzymatic processes not present in bacteria (e.g. the rabbit beta-globin gene which requires posttranscriptional RNA processing for expression), current knowledge of the subtle interactions of enzymes necessary for these controls is insufficient and renders bacterial expression systems unfeasible. For these reasons, eucaryotic expression systems are necessary.

For ease of growth and transformability with heterologous DNA sequences, the yeast Saccharomyces cerevisiae has been a eucaryotic system utilized for the expression of complex gene clusters and it has some advantages over the bacterial system. Many of the mRNA and protein processing systems present in mammalian cells are also present in yeast cells, but the specificity of the enzyme reactions is determined by different signals [Nature, 283, 835 (1980), Gene, 33, 215 (1985)]. More fundamentally, it is known that yeast cells utilize a different codon usage than mammalian cells, a fact which further complicates the correct expression of biologically active proteins from heterologous DNA templates [J. Biol. Chem., 257, 3026 (1982)]. If one can express a structurally correct mammalian protein product in yeast cells, it is often preferred that this protein be secreted from the cell to facilitate purification from the myriad of cell proteins present. The successful secretion of a peptide from yeast cells depends upon the size, glycosylation, conformation and solubility of the protein as well as some unidentified factors which together confound the predetermined control of these factors in establishing satisfactory secretion of heterologous proteins from these cells [MacKay, "Biological Research on Industrial Yeast", Vol. 1: Applied Aspects, Uniscience Series, CRC Press, Boca Raton (1987)]. Thus, although yeast offers a better system than bacteria for expression of mammalian proteins, it appears that the system of choice for such expression of therapeutically active mammalian proteins is mammalian cells.

The major obstacle to overcome in using mammalian cells as expression systems is the general inability of these cells to support expression of heterologous genes at the high levels necessary for pharmacological development. Limited success has been achieved by fusion of the SV40 origin-enhancer sequences upstream of specific genes and subsequent expression in COS-7 cells; however, expression in COS-7 cells can result in aberrant transcription of the heterologous RNA molecules [Eucaryotic Viral Vectors (ed. Y. Gluzman) pp. 61-66, Cold Spring Harbor Laboratory, Cold Spring Harbor, NY (1982)]. The introduction of SV40-enhancer-containing constructs into other cell lines such as HeLa cells results in correct transcription and processing of heterologous genes, but the DNA of these constructs lacks an origin of replication and thus cannot be stably maintained in the cell line as episomes. These technical problems are due in part to the restricted host range of SV40. The use of a gene expression system from a virus exhibiting a wider host range is certainly more desirable. Herpes Simplex Virus (HSV) replicates in a wide range of cell types and exhibits a defined control of genetic expression during various phases of its replicative cycle [Developments in Molecular Virology, Vol. VI, Viral mRNA: Transcription, Processing, Splicing, and Molecular Structure (ed. Y. Becker), Martinus Nijhoff: The Hague, pp 79-99 (1985)]. Transcriptional expression of herpesvirus genes occurs in three general phases: the immediate early (alpha), delayed early (beta) and late (gamma) periods. The major control of expression appears to be at the transcriptional level, and very little regulation is supplied by posttranscriptional processes.

One of the factors affecting selective initiation of HSV-1 alpha phase gene transcription is a transactivating virion component, the Vmw65 protein [J. Mol. Biol., 180:1-19 (1984)]. Transactivating virion components, including Vmw65, are produced by the host cell from the viral genetic material and stored in new virus particles. After invasion of a new host cell by these new virus particles, and after incorporation of viral nucleic acid sequences into the host genome, the transactivating components interact with certain regions of the viral sequences. This interaction stimulates transcription of the adjacent downstream genes, which usually code for proteins needed by the virus during the early phase (alpha) of its replication cycle. Optimal activation by Vmw65 requires specific DNA structural elements, including an activation-response DNA sequence corresponding to and recognized by the Vmw65 protein. This activation-response DNA is situated usually on or near the alpha phase promoter so that recognition of the activation-response DNA sequence by the transactivating Vmw65 protein stimulates transcription of the heterologous gene located downstream from the alpha phase promoter. A number of proteins in the Herpes Simplex Virus have similar transactivating potential when their corresponding activation-response DNA sequences are located upstream from alpha phase promoters. These proteins include the intercellular proteins ICP4, ICP0 and ICP27. Similar transactivating mechanisms exist, for example, in the Pseudorabies virus (PRV-IE gene product), Adenovirus (Ad-EIA) and Human cytomegalo-virus (HCMV-MIE protein).

Thus, we are proposing a system of protein expression which takes advantage of the interaction between viral transcriptional activating proteins and the corresponding activation-response DNA sequences that are influenced by these proteins. This system is different from other mammalian expression systems because 1) the levels of the transactivating protein can be manipulated by the investigator; 2) some specific DNA control sequences necessary for transactivation have been identified and can be used to control specific gene activation, rather than depending upon general effects of transcriptional activation used in other systems, and 3) this system should be applicable to a wide range of tissue culture cell types.

## SUMMARY OF THE INVENTION

This invention provides a stable cell line which has been stably transformed with a recombinant DNA molecule comprising the gene sequence for a viral transcriptional activating protein whereby said cell expresses said viral transcriptional activating factor.

The invention also provides a method for stimulating gene expression that comprises providing a viral transcriptional activating protein within a cell, said cell containing a recombinant DNA molecule and capable of stable replication, said DNA sequence comprising:

    a) a non-viral gene whose expression is desired;

    b) a promoter region preceding said gene; and

    c) a corresponding activation-response DNA sequence preceding said gene.

A preferred method for stimulating gene expression comprises providing the Herpes Simplex Virus I (HSV-1) stimulating factor, Vmw65, to a DNA sequence, said DNA sequence comprising:

    a) a non-viral gene whose expression is desired;

    b) a promoter region preceding said gene;

    c) the nucleotide sequence TAATGARAT, wherein R is either G or A, preceding said promoter region.

The existence of the HSV-1 stimulating factor Vmw65 is known in the art, Nucleic Acids Res., 13, 1876 (1985), but has not been described in association with stable cell lines. In the prior art, it has only been utilized in association with transient cell lines. The TAATGARAT activation-response DNA sequence (responsive to Vmw65) is described in Nucleic Acids Res. 14, 929 (1986). Other viral stimulatory factors are also known to exist, such as Ad-EIA [Proc. Nat. Acad. Sci. USA, 82, 381 (1985)], PRV-IE [Cell, 35, 137 (1983)], ICP0 [J. Virol., 49, 190 (1984)], ICP4 [Nature, 285, 329 (1980)] and ICP27 [J. Virol., 55, 796 (1985)]. The structures, sequences or precise mechanism of action of the activation-recognition sites for these stimulatory factors have not been fully elucidated.

For purposes of the invention, the preferred promoter regions are the alpha promoters of HSV-1. These promoters are described in J. Virol. 50, 708 (1984). The HSV-2 alpha promoters are also useful.

By practice of the present invention, enhancement of gene expression over that normally obtained in the absence of the activation-response DNA sequence and the corresponding transactivating stimulatory factor is obtained. Typical genes whose expression may be stimulated by the practice of the present invention encode human proteins, e.g., the interferons ($\alpha$, $\beta$ and $\gamma$), the interleukins (1,2,3,4 and 5), insulin, human growth hormone (HGH), the colony stimulating factors such as granulocyte colony stimulating factor (G-CSF) and granulocyte macrophage colony stimulating factor (GM-CSF), tissue plasminogen activator (TPA), erythropoietin (EPO), superovide dismutase (SOD) and the like. Modified human genes as well as non-human genes may also be utilized in the practice of the present invention but in any case, the gene whose expression is to be stimulated is always a non-viral gene.

The invention also contemplates the unique recombinant DNA constructs which comprise in sequence activation-response DNA sequence, followed by a promoter sequence, followed by a gene whose expression is desired.

In its preferred embodiment, the invention also contemplates the unique recombinant DNA constructs which comprise in sequence the TAATGARAT sequence, followed by a promoter sequence, followed by a gene whose expression is desired.

The invention contemplates stable cell lines which contain the above described recombinant DNA constructs. The stable cell lines contain a vector that is able to reproduce with the cell line. As such, we provide permanently transformed cell lines which are able to express indefinitely the protein encoded by the DNA that has been introduced. This invention contemplates producing such stable transformed cell lines.

Standard methods can be used for the preparation of the above-described recombinant DNA constructs and for transforming cells to contain such constructs. See, for example, Maniatis, T. et al., Molecular Cloning, A Laboratory Manual, Cold Spring Harbor Laboratory, USA (1982).

The following examples illustrate but do not limit the invention.

## Example 1

Plasmid clone PMGH [Nature, 300, 611 (1982)] was digested with Hind III and Bam HI restriction endonucleases and the excised fragment of rat growth hormone gene was separated from the remainder of the plasmid by gel electrophoresis in a 1 percent agarose matrix, according to established procedures. The vector portion of PMGH was eluted from the agarose and saved for ligation, using T4 DNA ligase, to the Hind III-Bam HI portion of plasmid pON 1 [J. Virol., 56, 135 (1985)], which contains the E. coli beta galactosidase (lac Z) gene and SV40 polyadenylation signals. After transfection into E. coli HB101 cells and selection with ampicillin, the resultant plasmid vector MTRGHβgal was recovered.

MTRGH βgal vector was digested with Xho I and Hind III to remove the remaining rat DNA sequences. The deleted region was replaced with the 6.1 Kilobase Xho I- Hind III fragment of HSV-1 DNA from the plasmid Hind III-Bgl II-DG [J. Virol., 43, 594 (1982)], which contains the coding sequences of the HSV-1 virion stimulatory protein (Vmw65). After transfection and selection, the resultant plasmid, MTXHADβgal, contained the Vmw65 coding region located downstream of the transcriptional enhancer/promoter region of the mouse metallothiorein gene.

Mouse L-M(TK-) (ATCC CCL 1.3) cells were cotransformed with 1 μg each of plasmid MTXHADβgal and plasmid pDG504 [J. Virol., 46, 1045 (1983)] DNA and 18 μg of sheared calf thymus DNA, using the calcium phosphate coprecipitation method [Virology, 52, 456 (1973)]. TK+ cells were selected, amplified, and maintained in Eagles Minimal Essential Medium containing 10 percent fetal calf serum and 1X hypoxanthine/aminopterin/thymidine (HAT) selective agents [Cell, 14, 725 (1979)]. Thymidine-kinase-positive cells which also contained the MTXHADβgal DNA were assayed by DNA dot blots of whole cell DNA [Proc. Natl. Acad. Sci. USA. 77, 5201 (1980)] and hybridization to $^{32}$P-labelled nick translated XHAD fragment DNA [J. Mol. Biol., 113, 237 (1977)]. Any cell that demonstrated hybridization to XHAD is XHAD+, while failure to demonstrate hybridization to XHAD results in designation as XHAD-. TK+ cells that possessed XHAD (referred to as TK+XHAD+ cells) DNA were amplified and the production of Vmw65 protein was assayed by the ability of these cells to activate gene expression from appropriate DNA control sequences.

## Example II

The ability of the TK+XHAD+ cells to express functional Vmw65 protein and transactivate heterologous gene transcription was tested using a transient gene expression assay developed by Geballe et al. [Cell, 46, 865, (1986)]. XHAD DNA possessing cell clones were plated in 96 well microtiter plates and grown to confluence (approximately 350000 cells). Each well was transfected with 0.5 μg of pON 105 DNA (obtained from E. Mocarski, Stanford University) by the DEAE Dextran method [J. Mol. Appl. Genet., 2, 101 (1983)]. pON 105 contains the bacterial beta galactosidase (lac Z) gene under the transcriptional control of the HSV-1 ICP4 promoter sequence, which is transcriptionally activated by functional Vmw65. 48 hours post-transfection, beta galactosidase activity was quantified by a fluorimetric assay. Levels of beta galactosidase activity in one subdose that was designated MTX5 approached the levels seen using a parallel transfection of TK+XHAD- cells with the pON 105 plasmid, followed by HSV-2 (strain MS) superinfection at 48 hours, at a Multiplicity of Infection of 5, and assay at 72 hours post transfection. Specificity of the Vmw65-ICP4 activator-promoter interaction was ascertained by transfection of XHAD+ cells with plasmid pON 245 DNA (obtained from E. Mocarski, Stanford University). pON 245 contains the lac Z gene under transcriptional control of the HSV-2 thymidine kinase promoter, which does not contain Vmw65 reactive elements. Assays using the pON 245 plasmid showed no induction of beta galactosidase activity by Vmw65. Induction of transactivating activity with pON 245 in the presence of superinfecting virus (+HSV in Table 1) demonstrated that the plasmid was intact and transcriptionally competent.

## Example III

The ability of the TK+XHAD+ cells to induce transcriptional activity from DNA sequences encoding transactivating viral proteins, other than ICP4, was tested by similar transient expression assays. Cells expressing Vmw65 protein were plated in 96 well microtiter plates and grown to confluence. Wells were transfected with 0.5 μg of pON 249 DNA [Cell 46, 865, (1986)], with 0.5 μg of pON 249 DNA and 0.5 μg of PRS1 DNA [Nucleic Acids Res., 15, 905 (1987)], or with 0.5 μg of pON 249 DNA and 0.5 ug of PES12 DNA (obtained from R. Stenberg, West Virginia University) by the DEAE dextran method. pON 249 contains the lac Z gene of E. coli under the transcriptional control of the major immediate early (MIE) promoter regulatory region of human cytomegalovirus (HCMV). PRS1 contains the HSV-1 ICPO gene with transcriptional regulatory regions known to respond to HSV-1 Vmw65 transactivation [Nucleic Acids Res., 14, 929 (1986)]. PES12 contains the HCMV-MIE gene and homologous promoter region, which does not exhibit a TAATGARAT homolog [Proc. Natl. Acad. Sci. USA, 81, 659 (1984)]. Both the ICPO and HCMV MIE proteins are known to stimulate transcriptional activity of heterologous viral promoter regions [J. Gen. Virol., 67, 2507 (1986); J. Virol., 49, 190 (1984)]. Despite the absence of a TAATGARAT homolog in the HCMV MIE promoter, increased levels of beta galactosidase activity were observed in cells transfected with pON 249 DNA. In cells cotransfected with either pON 249 and PRS1 DNAs or pON 249 and PES12 DNAs, levels of beta galactosidase are elevated even higher than those observed in cells transfected with only pON 249 DNA.

The results of Example III demonstrate that 1) sequences and/or DNA structure present in non-HSV promoter regions may respond to Vmw65 transactivation, 2) the Vmw65 transactivation can be mediated through DNA sequences not containing an apparent TAATGARAT homolog, and 3) Vmw65 transactivation can be used to induce synthesis of other transactivating gene products, which may augment the action of Vmw65 protein on heterologous promoter regions or act independently to transactivate heterologous genes present within the same cell.

The results of Examples II and III are summarized in Table 1.

TABLE 1

Table 1. Beta Galactosidase activity in transfected cells.

| Plasmid DNA transfected | Related beta galactosidase levels | | |
| | Cell line | | |
| | L TK– | MTX5 | MTX5 |
| | (–HSV) | (–HSV) | (+HSV) |
| pON 105 | 1 | 60 | 60 |
| pON 245 | 1 | 2 | 90 |
| pON 249 | 1 | 5.6 | 80 |
| pON 249, PRS1 | 1 | 16 | NT |
| pON 249, PES12 | 1 | 20 | NT |

NT = Not Tested

The above assays demonstrate that 1) the HSV-1 Vmw65 gene can be stably introduced into mammalian tissue culture, 2) the protein produced in tissue culture resembles biologically active Vmw65 in its ability to transactivate other HSV promoters, 3) heterologous (non-viral) protein production can be stimulated in these transformed cells if, and only if, the promoter sequences of the heterologous gene contain Vmw65 responsive elements known to function in the natural expression of HSV 1 genes, and 4) the levels of induction of transcriptional activity are roughly equivalent to those seen in the natural viral replicative cycle.

Given the high levels of transcriptional activity observed in HSV ICP4 gene expression, we feel that the application of this system to the production of pharmacologically important proteins will become an important alternative to current methods of producing heterologous proteins in cell culture systems.

The cloned gene or biologically active portion of the gene for any transactivating viral protein can be utilized in this system if 1) the activation-response DNA sequences are identified and 2) the relative orientation of these sequences to the promoter element of the responsive gene is maintained when substituting heterologous protein coding sequences for the original reading frame.

Any DNA sequence, natural or synthetic, which is responsive to the transactivating protein can be used to activate the expression of heterologous polypeptide from cloned DNA.

Cells which can be transformed, either by the calcium phosphate method or using viral vectors, may be any which can be cultured in vitro, such as human foreskin fibroblasts, human HeLa cells, mouse L cells, human KB cells, COS-7 cells, avian cells, reptilian cells, yeast cells and the like.

**Claims**

1. A stable cell line which has been stably transformed with a recombinant DNA molecule comprising the gene sequence for a viral transcriptional activating protein whereby said cell expresses said viral transcriptional activating factor.

2. A method for stimulating gene expression that comprises providing a viral transcriptional activating protein within a cell, said cell containing a recombinant DNA molecule and capable of stable replication, said recombinant DNA molecule comprising:

    a) a gene whose expression is desired;

    b) a promoter region preceding said gene;

    c) a corresponding activation-response DNA sequence preceding said gene.

3. A method for producing a polypeptide comprising culturing a cell which has been transformed with a recombinant DNA molecule in the presence of a transcriptional activating protein, said DNA molecule comprising:

    a) a non-viral gene which codes for said polypeptide;

    b) a promoter region preceding said gene; and

    c) an activation-response DNA sequence preceding said gene.

4. A stable cell line which has been stably transformed with:

    a) a first recombinant DNA molecule which comprises the gene sequence for a viral transcriptional activating protein whereby said cell expresses said viral transcriptional activating protein; and

    b) a second recombinant DNA molecule which comprises:

    1) a non-viral gene which codes for a desired protein;

    2) a promoter region preceding said gene; and

    3) a corresponding activation-response DNA sequence preceding said gene.

5. The methods and cells defined in claims 1, 2, 3 and 4 wherein said corresponding activation-response DNA sequence precedes said promoter region.

6. The methods and cells defined in claim 5 wherein said viral transcriptional activating protein is Vmw65, ICP4, ICP0 or ICP27.

7. The methods and cells defined in claim 6 wherein said viral transcriptional activating protein is Vmw65.

8. The methods and cells defined in claim 7 wherein said activation-response DNA sequence is TAATGARAT, wherein R is G or A.

9. The methods and cells defined in claim 8 wherein said gene is a heterologous non-viral gene.

10. The methods and cells defined in claim 9 wherein said cell has also been transformed with a recombinant DNA molecule which comprises the gene sequence which codes for Vmw65 whereby said cell expresses said Vmw65.

11. The methods and cells defined in claim 10 wherein said promoter region is chosen from the alpha HSV-1 and HSV-2 promoter regions.

12. The methods and cells defined in claim 5 wherein said gene is chosen from among the genes which code for the α-interferons, β-interferons, γ-interferons, interleukins 1, 2, 3, 4 or 5, G-CSF, GM-CSF, TPA, human insulin, EPO, HGH and SOD.